# EUROPEAN PATENT SPECIFICATION

(11) **EP 1 142 590 B1**
(45) Date of publication and mention of the grant of the patent: **01.10.2008**
(21) Application number: 00971697.8
(22) Date of filing: 26.10.2000
(51) Int. Cl.: A61K 48/00, A61K 38/22, A61K 9/127, A61P 9/10, A61P 3/10

(54) **GENE THERAPY FOR DIABETIC ISCHEMIC DISEASE**
GENTHERAPIE FÜR DIABETISCHE ISCHÄMIE
THERAPIE GENIQUE POUR TRAITER LES MALADIES ISCHEMIQUES DIABETIQUES

(30) Priority: 29.10.1999 JP 30998499
(43) Date of publication of application: 10.10.2001
(73) Proprietor: AnGes MG, Inc., Toyonaka-shi, Osaka (JP)
(72) Inventor: MORISHITA, Ryuichi, Osaka 565-0851 (JP); OGIHARA, Toshio, Minoo-shi, Osaka 562-0046 (JP)
(74) Representative: Grünecker, Kinkeldey, Stockmair & Schwanhäusser Anwaltssozietät
(86) International application number: PCT/JP2000/007502
(87) International publication number: WO 2001/032220

(56) References cited:
- WO-A-00/62798
- WO-A-98/19712
- WO-A-98/51798
- WO-A-99/36103
- WO-A1-97/14307
- WO-A1-99/36103
- UEDA HIDEKI ET AL: "In vivo gene transfection of hepatocyte growth factor attenuates ischemia-reperfusion injury in the heart: Evidence for a role of HGF in endogenous myocardial protection" CIRCULATION, vol. 96, no. 8 SUPPL., 1998, page I619, XP002935421 & 70TH SCIENTIFIC SESSIONS OF THE AMERICAN HEART ASSOCIATION; ORLANDO, FLORIDA, USA; NOVEMBER 9-12, 1997 ISSN: 0009-7322
- AOKI MOTOKUNI ET AL: "Beneficial angiogenesis induced by over-expression of human hepatocyte growth factor (HGF) in non-infarcted and infarcted myocardium: Potential gene therapy for myocardial infarction" CIRCULATION, vol. 98, no. 17 SUPPL., 27 October 1998 (1998-10-27), page I321, XP002936330 & 71ST SCIENTIFIC SESSIONS OF THE AMERICAN HEART ASSOCIATION; DALLAS, TEXAS, USA; NOVEMBER 8-11, 1998 ISSN: 0009-7322
- MORISHITA R ET AL: "Therapeutic angiogenesis induced by human recombinant hepatocyte growth factor in rabbit hind limb ischemia model as cytokine supplement therapy ." HYPERTENSION, (1999 JUN) 33 (6) 1379-84. JOURNAL CODE: 7906255. ISSN: 0194-911X., June 1999 (1999-06), XP002308376
- ERIC VAN BELLE ET AL.: 'Potentiated angiogenic effect of scatter factor/hepatocyte growth factor via induction of vascular endothelial growth factor' CIRCULATION vol. 97, 1998, pages 381 - 390, XP002936327
- GRAND D.S. ET AL.: 'Scatter factor induces blood vessel formation in vivo' PROC. NATL. ACAD. SCI. USA vol. 90, 1993, pages 1937 - 1941, XP002936328
- AOKI MOTOKUNI ET AL.: 'Beneficial angiogenesis induced by over-expression of human hepatocyte growth factor (HGF) in non-infarcted and infarcted myocardium: Potential gene therapy for myocardial infarction' CIRCULATION vol. 98, no. 17, 1998, page I321, XP002936329
- UEDA HIDEKI ET AL.: 'In vivo gene transfection of hepatocyte growth factor attenuates ischemia-reperfusion injury in the heart: Evidence for a role of HGF in endogenous myocardial protection' CIRCULATION vol. 96, no. 8, 1997, page I619, XP002936330

## Description

### Technical Field

The present invention relates to a gene therapy agent and gene therapy method for diabetic ischemic disease utilizing a hepatocyte growth factor (HGF) gene. More specifically, the present invention relates to a method of gene therapy for diabetic ischemic disease which comprises the noninvasive administration of therapeutic agents of diabetic ischemic disease comprising an HGF gene as the effective ingredient or HGF gene.

### Background Art

HGF is a protein that was first discovered as a strong growth factor for mature hepatocytes and the gene encoding it has been cloned (Biochem.Biophys.Res.Commun. 122, 1450 (1984); Proc.Natl.Acad.Sci.USA 83, 6489 (1986); FEBS Letter 22, 231 (1987); Nature 342, 440 (1989); Proc.Natl.Acad.Sci.USA 87, 3200 (1991)). Afterwards, according to researches, it has been revealed that HGF does not only work for repair and regeneration of the damaged liver, as a hepatocyte regeneration factor *in vivo*, but also has an angiogenic function and plays an important role in treatment and prevention of ischemic disease and artery disease (Symp.Soc.Exp.Biol. 47cell behavior, 227-234 (1993); Proc.Natl.Acad.Sci.USA 90, 1937-1941 (1993); Circulation 97, 381-390 (1998)). That is, it has been reported that upon administration of HGF to the rabbit lower limb ischemic model, significant angiogenesis is observed and improvement in blood flow, repression of blood pressure decrease and improvement in ischemic symptoms take place . According to these reports, it is believed today that HGF expresses and functions as one of the angiogenic factors.

As stated above, HGF has various functions to begin with functions as angiogenic factor, and many attempts have been made to use it as a drug. However, the half life of HGF in blood arose as a problem. The half life of HGF is as short as about 10 minutes, making it difficult to maintain its concentration in blood. Thus, problems arose as to how to deliver effective levels of HGF to the affected site.

Generally it is common knowledge that protein preparations are mostly administered intravenously and concerning the case above of HGF administration for the ischemic disease model, examples of intravenous and intra-arterial administration are shown (Circulation 97, 381-390 (1998)). In spite of the fact that effectiveness against ischemic disease or artery disease of intravenous or intra-arterial HGF administration to such animal models are revealed, specific administration methods, doses and so on effective for HGF are still under investigation. Particular effective administration methods or doses and such for HGF proteins are still to be determined, due to problems concerning its half life and delivery to the affected site described above.

On the other hand, the rapid progress lately in molecular biology has made it possible to activate cellular function by gene transfer methods and various attempts have been made. Some trials have been made for gene therapy of the heart region. There are some methods, like the coronary diffusional infusion method and such, reported for gene transfer methods but there is no case of gene transfer methods to the ischemic site, particularly intramuscular infusion method to the skeletal muscle showing effects on specific diabetic ischemic disease.

Further, it is known that angiogenesis hardly occurs and prognosis is unfavorable in ischemic disease complicated with or caused by diabetes. At present, it is not known whether HGF gene administration to such diabetic ischemic disease is effective or not.

### Disclosure of the Invention

The object of this invention is to provide therapeutic agents and treatment methods for diabetic ischemic disease that utilize the HGF gene.

Inventors investigated to find out whether the HGF gene can be adapted to diabetic ischemic disease and revealed that extremely effective results are obtained by administering HGF gene directly to the ischemic affected site. Specifically, relating to lower limb ischemic disease, it was found out that effective results are obtained by administering HGF gene to the lower limb layer . As mentioned above , it is known that angiogenesis hardly occurs and prognosis is unfavorable in ischemic disease complicated with or caused by diabetes. Therefore, unlike mere ischemic disease, it had been unknown whether the HGF gene is effective toward diabetic ischemic disease. This invention revealed the effectiveness of the HGF gene for diabetic ischemic disease for the first time.

Since this method is a noninvasive treatment, it has the advantage that it is possible to administer the present gene repeatedly according to the condition.

Thus, the outline of the present invention is as follows:
(1) a therapeutic agent for diabetic ischemic disease, which comprises hepatocyte growth factor (HGF) as the effective ingredient;
(2) the therapeutic agent according to (1), used for administration to the ischemic site;
(3) the therapeutic agent according to (1) or (2), wherein the diabetic ischemic disease is selected from the group consisting of diabetic lower limb ischemic disease, diabetic ischemic neuropathy or diabetic ischemic myocardial infarction;
(4) the therapeutic agent according to (3), wherein the diabetic ischemic disease is diabetic lower limb ischemic disease;
(5) the therapeutic agent according to any of (1) to (4), used for administration into the muscle of the ischemic site;
(6) the therapeutic agent according to any of (1) to (5), wherein the HGF gene is in the form of a Sendai virus (HVJ)-liposome;
(7) the therapeutic agent according to any of (1) to (6), which is to be administered repeatedly as needed;
(8) the therapeutic agent according to any of (1) to (7), wherein the amount of HGF gene used is at least 50 µg;
(9) a method for the treatment of diabetic ischemic disease, which comprises the transfer of the HGF gene into human;
(10) the method according to (9), wherein the HGF gene is administered to an ischemic site;
(11) the method according to (9) or (10), wherein the diabetic ischemic disease is selected from the group consisting of diabetic lower limb ischemic disease, diabetic ischemic neuropathy or diabetic ischemic myocardial infarction;
(12) the method according to (11), wherein the diabetic ischemic disease is diabetic lower limb ischemic disease;
(13) the method according to any of (9) to (12), wherein the HGF gene is administered into the muscle of ischemic site;
(14) the method according to any of (9) to (13), wherein the HGF gene is in the form of a Sendai virus (HVJ)-liposome;
(15) the method according to any of (9) to (14), wherein the HGF gene is administered repeatedly as needed;
(16) the method according to any of (9) to (15), wherein the amount of HGF gene to be administered is at least 50 µg;
(17) use of the HGF gene for preparing therapeutic agents for diabetic ischemic disease;
(18) the use according to (17), wherein the diabetic ischemic disease is selected from the group consisting of diabetic lower limb ischemic disease, diabetic ischemic neuropathy or diabetic ischemic myocardial infarction;
(19) the use according to (18), wherein the diabetic ischemic disease is diabetic lower limb ischemic disease;
(20) the use according to any of (17) to (19), wherein the HGF gene is in the form of a Sendai virus (HVJ)-liposome; and
(21) the use according to any of (17) to (20), wherein the amount of HGF gene to be used is at least 50 µg.

### Brief Description of the Drawings

Figure 1 is a graph showing changes in blood perfusion ratio over time of the group of rats with diabetic lower limb ischemia in reference 1 and of the control group, in which lower limb ischemia was induced in normal rats.
Figure 2 is a graph showing the internal HGF concentration in ischemic muscle of the group of rats with diabetic lower limb ischemia in reference 1 and of the control group, in which lower limb ischemia was induced in normal rats.
Figure 3 shows the the blood perfusion ratio of the group of rats with diabetic lower limb ischemia in reference 1, to which HGF gene was administered or not, and of the control group, in which lower limb ischemia was induced in normal rats.
Figure 4 is a graph showing the result of a comparison of the number of blood vessels of the group of rats with diabetic lower limb ischemia in reference 1, to which HGF gene was administered or not, and of the control group, in which lower limb ischemia was induced in normal rats by ALP (alkalinephosphatase) staining of the skeletal muscle of the lower limb ischemic site.
Figure 5 is a graph showing the MMP-1 concentration in the culture supernatant of the glucose added angioendothelial cell in reference 2, to which HGF was added or not, and of the control group, to which no glucose was added.
Figure 6 is a graph showing the amount of mRNA of transcription factor which is expressed in the angioendothelial cell, of the group of glucose added angioendothelial cell in reference 3, to which HGF was added or not, and of the control group to which no glucose was added.

### Best Mode for Carrying out the Invention

As used herein "HGF gene" means a gene that can express HGF (the HGF protein). Specifically, cDNA of HGF described in Nature 342: 440 (1989); Japanese Patent Publication No., 2777678; Biochem.Biophys.Res.Commun. 163: 967 (1989); and Biochem.Biophys.Res.Commun. 172: 321 (1990) and so on integrated into suitable expression vectors (non-viral vector, viral vector) described below are to be mentioned. The base sequence of the cDNA encoding HGF gene of the present invention has been described in the above literature and is also registered with databases such as GenBank. Thus, based on such sequence information, using a suitable DNA portion as a PCR primer, it is possible to clone the cDNA of HGF, for example, by performing a RT-PCR reaction on mRNA derived from the liver or leukocytes. Such cloning can easily be performed by a person skilled in the art according to a basic textbook, such as Molecular Cloning 2nd Ed., Cold Spring Harbor Laboratory Press (1989).

The HGF genes of the present invention are not restricted to the above mentioned genes but also include those genes that express proteins with substantially the same function as HGF. That is, the following genes fall under the category of the HGF gene of the present invention: 1) DNA that hybridize to said cDNA under stringent conditions, and 2) DNA encoding proteins having amino acid sequence in which 1 or more (preferably a few) amino acids are substituted, deleted and/or added to the protein encoded by said cDNA and which encodes proteins having the function as HGF. Above DNAs of 1) and 2) can be readily obtained, for example, by site-directed mutagenesis, PCR method, ordinal hybridization method and so on. Such methods can be easily accomplished according to the above basic textbook.

Subsequently, methods of gene transfer, dosage forms, doses and the like for use in gene therapy of the present invention are explained.

The dosage form of a gene therapy agent comprising the above gene as the effective ingredient to be administered to patients are roughly classified into two groups: one is the case in which a nonviral vector is used, and the other is in which a viral vector is used. Methods for preparation and administration thereof are explained in detail in experimental manuals (Supplement of Experimental Medicine, Basic Technology in gene therapy, Yodosha (1996); Supplement of Experimental Medicine, Experimental Methods in Gene Introduction and Expression Analysis, Yodosha (1997); Handbook for Development and Research of Gene Therapy, Japan Society of Gene Therapy ed., NTS (1999)). Specifics are explained below.

### A. Usage of a nonviral vector

Using a recombinant expression vector in which the gene of interest has been integrated into a commonly used gene expression vector, may be used to introduce the gene of interest into cells or tissue by the following method etc.

Illustrative methods of gene transfer into cells include the lipofection method, calcium phosphate co-precipitation method, DEAE-dextran method, direct DNA introduction methods using micro glass tubes, and the like.

Regarding methods of gene transfer into the tissue, a recombinant expression vector may be incorporated into the cell by subjecting it to any of the method, such as the method of gene transfer with internal type liposome, method of gene introduction with electrostatic type liposome, HVJ-liposome method, improved HVJ-liposome method (HVJ-AVE liposome method), receptor-mediated gene introduction method, method of introducing DNA molecules together with carriers (metal particles) by a particle gun, method of directly introducing naked-DNA, method of introduction with positively-charged polymers and the like.

Among them, HVJ-liposome is a fusion product prepared by enclosing DNA into liposome made of lipid bilayer, which is fused to inactivated Sendai virus (Hemagglutinating virus of Japan: HVJ). The HVJ-liposome method is characterized by a very high fusing activity with the cell membrane as compared to the conventional liposome method, and is a preferred mode of introduction. For the method of preparing HVJ-liposome, see the literature for details (Separate volume of Experimental Medicine, Basic Technology in gene therapy, Yodosha (1996); experimental Methods in Gene Introduction and Expression Analysis, Yodosha (1997); J.Clin.Invest. 93:1458-1464(1994); Am.J.Physiol. 271:R1212-1220(1996)) and the like, and experimental examples described below for details. In particular, the Z strain (available from ATCC) is preferred as the HVJ strain, but other HVJ strains (for example, ATCC VR-907 and ATCC VR-105) may also be used.

Furthermore, the method of directly introducing naked-DNA is the most simple method among the methods described above, and in this regard a preferred method of introduction. ,

Expression vectors as used herein may be any expression vectors so long as they permit the *in vivo* expression of the gene of interest. Examples include expression vectors such as pCAGGS (Gene 108:193-200(1991)), pBK-CMV, pcDNA3.1, pZeoSV (Invitrogen, Stratagene) and the like.

### B. Usage of a viral vector

Representativemethods that use viral vectors include those using viral vectors such as recombinant adenovirus, retrovirus and the like. More specifically, the gene of interest can be introduced into a DNA virus such as detoxified retrovirus, adenovirus, adeno-associated virus, herpes virus, vaccinia virus, poxvirus, poliovirus, Sindbis virus, Sendai virus, SV40, human immunodeficiency virus (HIV) and the like, which is then infected to the cell to introduce the gene into the cell.

Among the above viral vectors, the efficiency of infection of adenovirus is known to be much higher than that of other viral vectors . In this regard, it is preferred to use an adenovirus vector system.

As methods of introducing a gene therapy agent into a patient, there are *in vivo* methods, which permit direct introduction of the gene therapy agent into the body, and *ex vivo* methods, in which certain cells are removed from a human, wherein the gene therapy agent is introduced and which are then returned into the body (Nikkei Science, April 1994 issue pp.20-24; Monthly Yakuji, 36(1):23-48 (1994); Supplement To Experimental Medicine 12(15) (1994); Handbook for Development and Research of Gene Therapy, NTS (1999)). According to the present invention, the *in vivo* method is preferred.

Dosage forms may take various forms according to various administration regimens described above (for example, liquids). When, for example, an injection containing the gene as the effective ingredient is to be used, said injection may be prepared by dissolving the effective ingredients into a standard solvent (a buffer such as PBS, physiological saline, sterile water, etc.). The inj ection liquid may then be filter-sterilized with filter as needed, and then filled into sterilized containers. Conventional carriers and so on may be added to the injection. Liposomes, such as HVJ-liposome may take the form of suspensions, frozen formulations, centrifugation-concentrated frozen formulations and the like.

It is possible to use known factors having angiogenic functions, additionally or alone besides the HGF gene used in this invention. For example, it is reported that factors such as VEGF and FGF have an angiogenic function and therefore such genes can be used. Further, growth factors such as EGF are reported to repair cell damage in various tissues and thus genes encoding them can be also used.

The diabetic ischemic disease herein includes diseases such as diabetic lower limb ischemic disease, diabetic ischemic neuropathy and diabetic ischemic cardiac infarction and so on, and the therapeutic agent of this invention can be applied to any of these diseases. Moreover, the therapeutic agent of this invention can be applied not only to patients with critical diabetic ischemic disease but also to patients with progressively mild symptoms.

Proper methods and sites for administration adequate for the disease or symptom to be treated are selected for the gene therapy agent of this invention. As to the administration methods, parenteral administration methods are preferred. As a preferable administration site, the ischemic site can be mentioned. "Ischemic site" herein refers to the site including the affected site of ischemia and surrounding sites thereof.

Specifically, it is possible to administer into the blood vessel or into the muscle of the ischemic site. However, administration into the muscle of the ischemic site is preferred. In other words, administration into the skeletal muscle of the lower limb ischemic site enables stimulation of angiogenesis in the affected site of ischemia and improvement of blood flow. Thereby, it enables recovery and normalization of the function of the ischemic site. While in cardiopathy, such as cardiac infarction, it is possible to gain similar effect by administering into the cardiac muscle.

Examples of preferred administration methods include, for example, administration by noninvasive catheter, noninvasive injector and so on. Moreover, administration methods which utilize a noninvasive catheter, noninvasive injector and such under the usage of echo can be mentioned. As a method using noninvasive catheter, for example, injecting HGF genes directly into the cardiac muscle from the ventricle inner space in a cardiopathy can be indicated.

Application of the HGF gene of the present invention makes positive treatment for patients with diabetic ischemic disease possible. For example, it enables the recoveryof function inpatients with critical symptoms to whom no option, other than surgical excision of the affected site, is left.

Dosage of the therapeutic agent of this invention varies depending on the symptoms of the patient but HGF genes about 1 µg to about 50 mg, preferably about 10 µg to about 5 mg, more preferably about 50 µg to about 5 mg per adult patients can be defined.

The therapeutic agent of this invention is suited for administration once every few days or once every few weeks, and the frequency of administration is appropriately selected depending on symptoms of patients.

According to the therapeutic treatment of the invention, genes are administered noninvasively and, therefore, desired genes can be administered as much as the condition demands.

The present invention will now be specifically explained with reference to the following examples. It should be noted, however, that the present invention is not limited by these examples in any way.

### Materials and Methods

### Preparation of HVJ-liposome agent

10 mg dried lipid (a 1:4.8:2 mixture of phosphatidyl serine, phosphatidyl choline and cholesterol) and 200 µl balanced salt solution (137 µM NaCl, 5.4 µM KCl, 10 µM Tris-HCl; pH7.6) containing HGF gene (100 µg) -HMG1 (high mobility group 1 nuclear protein, 25 µg) was mixed and, by stirring vigorously with ultrasonication, liposomes were formed. Purified Sendai virus (Z strain) was irradiated with UV (110erg/mm²/sec) for 3 minutes. Liposome suspension was mixed with Sendai virus (HVJ), heated at 4°C for 10 minutes, and then heated at 37°C for 30 minutes. Free HVJ was discarded and thus obtained HVJ liposome agent.

### Experimental Animals

Administered group: ischemic rat of diabetic rat (DM-rat)
Control rat: ischemic rat of normal rat

### Administration method of HVJ-liposome agent

By surgically excising the femoral artery of one leg of the diabetic rat (6 weeks old; 6 animals per group), to which diabetes was provoked by interperitoneal administration of streptozotocin, ischemic state in the lower limb site was produced.

HVJ-liposome preparation containing HGF gene was injected to the lower limb skeletal muscle.

### Details of examination

After administration of the liposome preparation, the blood flow of the lower limb was measured by laser Doppler imager (LDI) using laser scatter analysis as the index of bypass circulation formation and effects of improvement in blood flow. The average of colored histogram of ischemic lower limb to that of the normal lower limb was taken as the perfusion ratio.

The density of blood capillary in the lower limb ischemic site was measured by alkalinephosphatase (ALP) staining, and the result of diabetic lower limb ischemic rat group was compared to that of the control lower limb ischemic rat group. Alternatively, comparison between the groups to which HGF gene was administered and to which no HGF gene was administered was made.

### Reference 1

### HGF expression state in the lower limb ischemic site of the diabetic rat

Ischemic state in the lower limb site was produced by surgical excision of the femoral artery of one leg of the diabetic rats (6 weeks old; 6 animals per group), to which diabetes was provoked by interperitoneal administration of streptozotocin, and of normal rats (6 weeks old; 6 animals per group) as the control group.

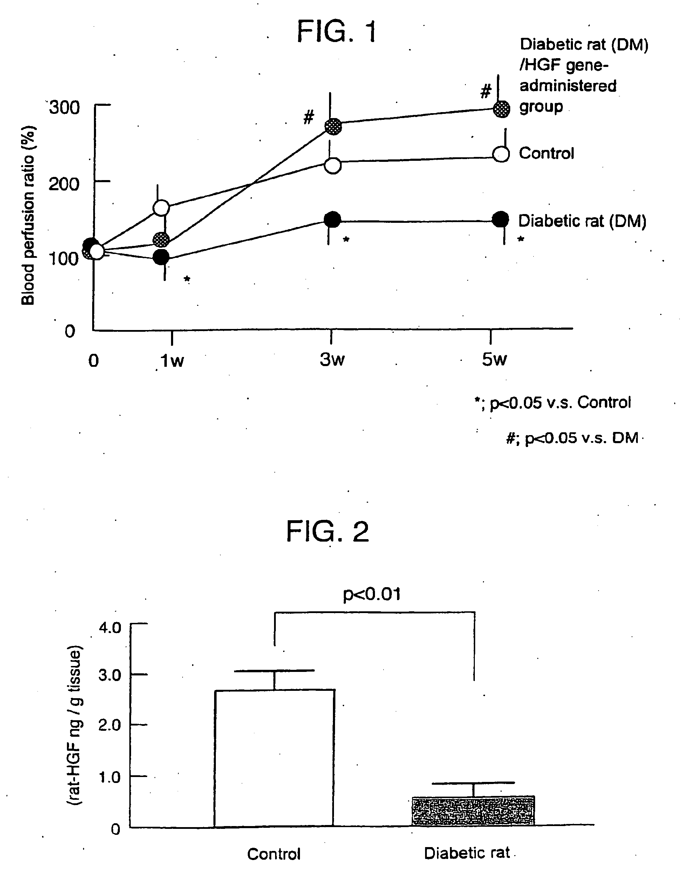

After one week, the perfusion ratio of the ischemic site was measured by laser Doppler imager. The perfusion ratio of the ischemic site or the diabetic lower limb ischemic rat was much lower than that of the control lower limb ischemic rat (see Figure 1).

The perfusion ratio of the lower limb was measured again 3 weeks and 5 weeks later, and the same results were obtained. That is, the perfusion ratio of the lower limb of the diabetic lower limb ischemic rat was much lower than that of the control lower limb rat (see Figure 1).

The internal HGF concentration in the muscles was significantly lower in the muscles of the ischemic site of the diabetic lower limb ischemic rat than that of the control lower limb ischemic rat. This result indicates that angiogenesis in diabetes is poor due to the decrease of internal HGF in the muscles. Therefore, angiogenesis hardly occurs in diabetic lower limb ischemic rat and bypass circulation does not develop (see Figure 2).

### Experiment 1

### Effect of HGF gene therapy against diabetic lower limb ischemic rat (I)

Ischemic state in the lower limb site was produced by surgical excision of the femoral artery of one leg of the diabetic rats (6 weeks old; 6 animals per group), to which diabetes was provoked by interperitoneal administration of streptozotocin. After surgical excision of the femoral artery, infusion of HVJ-liposome preparation containing HGF gene (50 µg) was injected into the muscle of the lower limb ischemic site.

After 3 weeks, the perfusion ratio of the ischemic site was measured by laser Doppler imager. The perfusion ratio of the ischemic site of the diabetic lower limb ischemic rat, to which HGF gene was administered, showed significant increase compared to that of the control lower limb ischemic rat or that of the diabetic lower limb ischemic rat above with no administration.

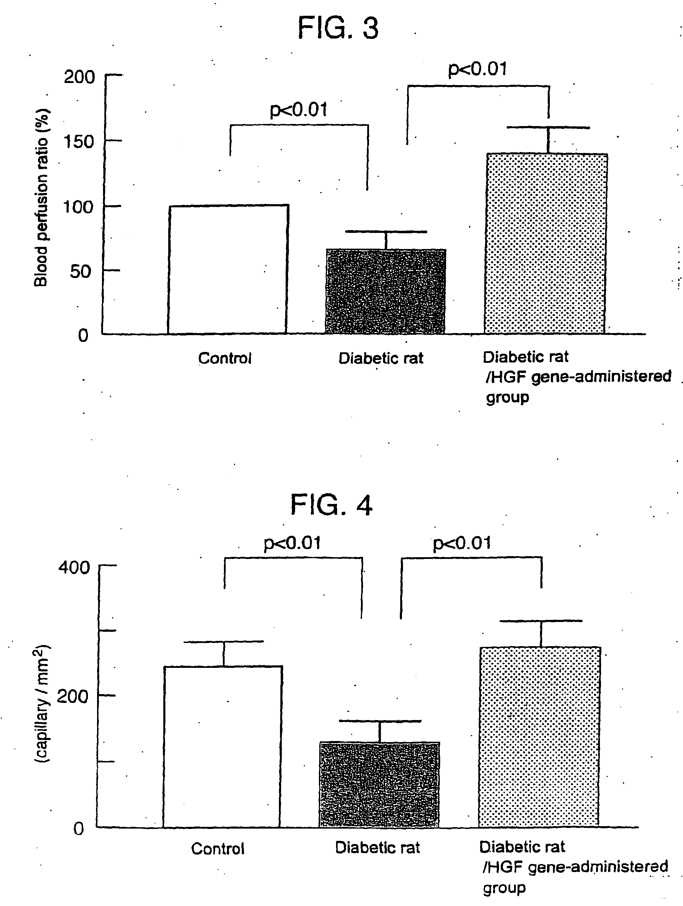

Taking perfusion ration of the control lower limb ischemic rat as 100%, that of the HGF gene untreated diabetic lower limb ischemic rat was 67% and that of the HGF administered diabetic lower limb ischemic rat was 129%. The results are shown in Figure 3 (see Figure 1).

### Experiment 2

### Effect of HGF gene therapy against diabetic lower limb ischemic rat (II)

Diabetic lower ischemic rat and control lower limb ischemic rat treated as above were prepared and subjected to HGF gene therapy. 5 weeks later, the skeletal muscle of the lower limb ischemic site was taken from each animal, subjected to ALP staining and the blood vessel count per unit area was compared. The blood vessel count of HGF gene untreated diabetic lower limb ischemic rat was significantly smaller as that of the control lower limb ischemic rat, and that of the HGF administered diabetic lower limb ischemic rat was significantly increased. The results are shown in Figure 4.

### Reference 2

### Influence of glucose concentration and HGF addition against MMP-1 production of the angioendothelial cell

The angioendothelial cells (derived from human aorta) were cultured in three types of serum free medium containing glucose at a concentration of 0, 25 mM and 50 mM, respectively. After 24 hours of cultivation, the MMP-1 concentration in the supernatant of the culture media was measured.

Each sample was compared to that in which 100 ng/ml of HGF was added 30 minutes before glucose addition.

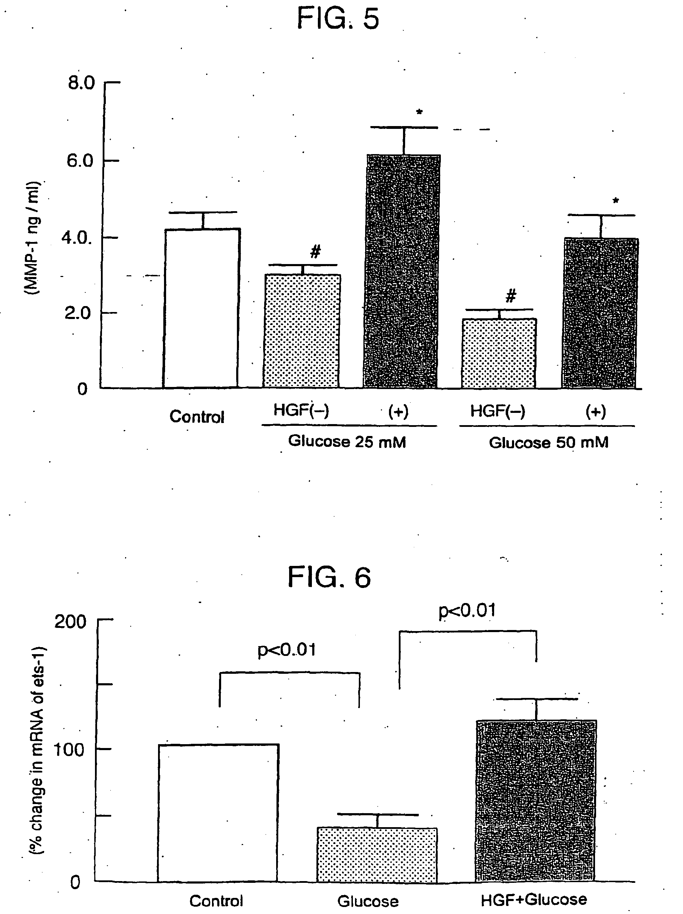

The MMP-1 concentration of the supernatant decreased significantly depending on the glucose concentration, and it was shown that decrease of MMP-1 was inhibited by HGF treatment. The results are shown in Figure 5.

### Reference 3

### Effect of HGF against angioendothelial cells (Changes of transcription factor ETS-1 related to angiogenesis)

Cultivation of anigoendothelial cell was conducted as in reference 2, and expression of mRNA of the transcription factor ETS-1 in the cell was detected. Taking the mRNA of ETS-1 in the control endothelial cell as 100 %, that of the HGF untreated angioendothelial cell decreased in a glucose dependent manner. On the other hand, HGF treated angioendothelial cells expressed the mRNA of ETS-1 at the same or more level compared to that of the control group (P<0.01). The results are shown in Figure 6.

As described above, angioendothelial cells under high glucose concentration show a decrease in MMP-1 expression, which is a matrix cleaving enzyme essential for angiogenesis, and show a decrease in the expression of mRNA of the transcription factor ETS-1, which is expressed and increases during angiogenesis.

Consequently, it was revealed that angiogenesis hardly occurs under high glucose condition. On the other hand, it was shown that by adding HGF to the angioendothelial cell under high glucose condition, MMP-1 expression and mRNA of ETS-1 expression increases significantly. Thus, it was revealed that HGF makes anigogenesis easier.

### Industrial Applicability

The therapeutic agent for diabetic ischemic disease containing an HGF gene as the effective ingredient improves poor angiogenesis specific to the affected site of diabetic ischemia with decrease in HGF expression and shows significant angiogenic effect. Therefore, it enables the improvement of the condition by increasing the blood flow in the affected site of ischemia. Moreover, the therapeutic agent of this invention can be administered more than once, depending on the symptoms of the patient, thereby stimulating angiogenesis. Therefore, according to these effects, the therapeutic agent of this invention makes it possible to treat diabetic lower limb ischemic disease, diabetic ischemic neuropathy and diabetic cardiac infarction.

## Claims

1. Use of a HGF gene for the preparation of a medicament for the treatment of a diabetic ischemic disease except for diabetic neuropathy.

2. The use according to claim 1, wherein the HGF gene is for administration to a human.

3. The use according to claim 2, wherein the medicament is prepared for administration of about 50µg to about 5mg of the HGF gene.

4. The use according to any of claims 1 to 3, wherein the HGF gene is for administration to an ischemic site or surrounding parts thereof.

5. The use according any of claims 1 to 4, wherein the diabetic ischemic disease is diabetic lower limb ischemic disease or diabetic ischemic myocardial infarction.

6. The use according to claim 5, wherein the diabetic ischemic disease is diabetic lower limb ischemic disease.

7. The use according to claim 6, wherein the HGF gene is for administration to skeletal muscle.

8. The use according to any of claims 1 to 7, wherein the HGF gene is in the form of a Sendai virus (HVJ)-liposome.

9. The use according to any of claims 1 to 8, wherein the HGF gene is for repeated administration as needed.

10. The use according to any of claims 1-9, wherein the HGF gene increases the mRNA level of ETS-1.

11. A HGF gene for treating a diabetic ischemic disease except for diabetic neuropathy.

12. The HGF gene according to claim 11, wherein the HGF gene is for administration to a human.

13. The HGF gene according to claim 12, wherein the HGF gene is for administration at a dosage of about 50µg to about 5mg.

14. The HGF gene according to any of claims 11 to 13, wherein the HGF gene is for administration to an ischemic site or surrounding parts thereof.

15. The HGF gene according any of claims 11 to 14, wherein the diabetic ischemic disease is diabetic lower limb ischemic disease or diabetic ischemic myocardial infarction.

16. The HGF gene according to claim 15, wherein the diabetic ischemic disease is diabetic lower limb ischemic disease.

17. The HGF gene according to claim 16, wherein the HGF gene is for administration to skeletal muscle.

18. The HGF gene according to any of claims 11 to 17, wherein the HGF gene is in the form of a Sendai virus (HVJ)-liposome.

19. The HGF gene according to any of claims 11 to 18, wherein the HGF gene is for repeated administration as needed.

20. The HGF gene according to any of claims 11-19, wherein the HGF gene increases the mRNA level of ETS-1.

## Patentansprüche

1. Verwendung eines HGF-Gens für die Herstellung eines Medikamentes zur Behandlung einer diabetisch ischämischen Krankheit außer diabetischer Neuropathie.

2. Die Verwendung gemäß Anspruch 1, worin das HGF-Gen zur Verabreichung an einen Menschen vorgesehen ist.

3. Die Verwendung gemäß Anspruch 2, worin das Medikament zur Verabreichung des HGF-Gens in einer Dosierung von etwa 50 µg bis etwa 5 mg vorgesehen ist.

4. Die Verwendung gemäß einem der Ansprüche 1 bis 3, worin das HGF-Gen zur Verabreichung an eine ischämische Stelle oder umgebende Stellen hiervon vorgesehen ist.

5. Die Verwendung gemäß einem der Ansprüche 1 bis 4, worin die diabetisch ischämische Krankheit eine diabetische ischämische Krankheit der unteren Gliedmaßen oder ein diabetisch ischämischer Herzinfarkt ist.

6. Die Verwendung gemäß Anspruch 5, worin die diabetisch ischämische Krankheit eine diabetische ischämische Krankheit der unteren Gliedmaßen ist.

7. Die Verwendung gemäß Anspruch 6, worin das HGF-Gen zur Verabreichung in den Skelettmuskel vorgesehen ist.

8. Die Verwendung gemäß einem der Ansprüche 1 bis 7, worin das HGF-Gen in Form eines Sendai-Virus (HVJ)-Liposoms vorliegt.

9. Die Verwendung gemäß einem der Ansprüche 1 bis 8, worin das HGF-Gen nach Bedarf zur wiederholten Verabreichung vorgesehen ist.

10. Die Verwendung gemäß einem der Ansprüche 1 bis 9, worin das HGF-Gen den mRNA Pegel von ETS-1 erhöht.

11. Ein HGF-Gen zur Behandlung einer diabetisch ischämischen Krankheit außer diabetischer Neuropathie.

12. Das HGF-Gen gemäß Anspruch 11, worin das HGF-Gen zur Verabreichung an einen Menschen vorgesehen ist.

13. Das HGF-Gen gemäß Anspruch 12, worin das HGF-Gen zur Verabreichung in einer Dosierung von etwa 50 µg bis etwa 5 mg vorgesehen ist.

14. Das HGF-Gen gemäß einem der Ansprüche 11 bis 13, worin das HGF-Gen zur Verabreichung an eine ischämische Stelle oder umgebende Stellen hiervon vorgesehen ist.

15. Das HGF-Gen gemäß einem der Ansprüche 11 bis 14, worin die diabetisch ischämische Krankheit eine diabetische ischämische Krankheit der unteren Gliedmaßen oder ein diabetisch ischämischer Herzinfarkt ist.

16. Das HGF-Gen gemäß Anspruch 15, worin die diabetisch ischämische Krankheit eine diabetische ischämische Krankheit der unteren Gliedmaßen ist.

17. Das HGF-Gen gemäß Anspruch 16, worin das HGF-Gen zur Verabreichung in den Skelettmuskel vorgesehen ist.

18. Das HGF-Gen gemäß einem der Ansprüche 11 bis 17, worin das HGF-Gen in Form eines Sendai-Virus (HVJ)-Liposoms vorliegt.

19. Das HGF-Gen gemäß einem der Ansprüche 11 bis 18, worin das HGF-Gen nach Bedarf zur wiederholten Verabreichung vorgesehen ist.

20. Das HGF-Gen gemäß einem der Ansprüche 11 bis 19, worin das HGF-Gen den mRNA Pegel von ETS-1 erhöht.

## Revendications

1. Utilisation d'un gène HGF pour la préparation d'un médicament destiné au traitement d'une maladie ischémique diabétique excepté la neuropathie diabétique.

2. Utilisation selon la revendication 1, dans laquelle le gène HGF est destiné à une administration à un humain.

3. Utilisation selon la revendication 2, dans laquelle le médicament est préparé pour une administration d'environ 50 µg à environ 5 mg du gène HGF.

4. Utilisation selon l'une quelconque des revendications 1 à 3, dans laquelle le gène HGF est destiné à une administration à un site ischémique ou à des parties entourant celui-ci.

5. Utilisation selon l'une quelconque des revendications 1 à 4, dans laquelle la maladie ischémique diabétique est une maladie ischémique diabétique des membres inférieurs ou un infarctus du myocarde ischémique diabétique.

6. Utilisation selon la revendication 5, dans laquelle la maladie ischémique diabétique est une maladie ischémique diabétique des membres inférieurs.

7. Utilisation selon la revendication 6, dans laquelle le gène HGF est destiné à une administration au muscle squelettique.

8. Utilisation selon l'une quelconque des revendications 1 à 7, dans laquelle le gène HGF se trouve sous la forme d'un liposome-virus de Sendai (HVJ).

9. Utilisation selon l'une quelconque des revendications 1 à 8, dans laquelle le gène HGF est destiné à une administration répétée si nécessaire.

10. Utilisation selon l'une quelconque des revendications 1 à 9, dans laquelle le gène HGF augmente les taux d'ARNm de ETS-1.

11. Gène HGF pour le traitement d'une maladie ischémique diabétique excepté la neuropathie diabétique.

12. Gène HGF selon la revendication 11, dans lequel le gène HGF est destiné à une administration à un humain.

13. Gène HGF selon la revendication 12, dans lequel le gène HGF est destiné à une administration à une posologie d'environ 50 µg à environ 5 mg.

14. Gène HGF selon l'une quelconque des revendications 11 à 13, dans lequel le gène HGF est destiné à une administration à un site ischémique ou à des parties entourant celui-ci.

15. Gène HGF selon l'une quelconque des revendications 11 à 14, dans lequel la maladie ischémique diabétique est une maladie ischémique diabétique des membres inférieurs ou un infarctus du myocarde ischémique diabétique.

16. Gène HGF selon la revendication 15, dans lequel la maladie ischémique diabétique est une maladie ischémique diabétique des membres inférieurs.

17. Gène HGF selon la revendication 16, dans lequel le gène HGF est destiné à une administration au muscle squelettique.

18. Gène HGF selon l'une quelconque des revendications 11 à 17, dans lequel le gène HGF se trouve sous la forme d'un liposome-virus de Sendai (HVJ).

19. Gène HGF selon l'une quelconque des revendications 11 à 18, dans lequel le gène HGF est destiné à une administration répétée si nécessaire.

20. Gène HGF selon l'une quelconque des revendications 11 à 19, dans lequel le gène HGF augmente le taux d'ARNm de ETS-1.
